# EUROPEAN PATENT APPLICATION

(11) **EP 4 635 489 A1**
(43) Date of publication of application: **22.10.2025**
(21) Application number: 23903677.5
(22) Date of filing: 04.09.2023
(51) Int. Cl.: A61K 31/198, A61K 31/375, A61K 31/385, A23L 33/10

(54) **COMPOSITION FOR PREVENTING OR TREATING SIDE EFFECTS OF CHOLINESTERASE INHIBITORS**

(30) Priority: 16.12.2022 KR 20220177363
(71) Applicant: Dr.Noah Biotech Inc., Suwon-si, Gyeonggi-do 16229 (KR)
(72) Inventor: LEE, Ji Hyun, Suwon-si, Gyeonggi-do 16229 (KR); KIM, Eun Jung, Suwon-si, Gyeonggi-do 16229 (KR)
(74) Representative: Richardt Patentanwälte PartG mbB
(86) International application number: PCT/KR2023/013147
(87) International publication number: WO 2024/128473

(57) **Abstract**

The present invention relates to a composition for preventing or treating an adverse event of cholinesterase inhibitors, more specifically, it relates to a composition comprising as an active ingredient an antioxidant capable of prevention, alleviation or treatment an adverse event caused by the administration of cholinesterase in a therapeutically effective amount or more.

## Description

### TECHNICAL FIELD

This application claims priority to South Korean Patent Application No. 10-2022-0177363, filed Dec. 16, 2022, the entirety of which is hereby incorporated by reference.

The present invention relates to a composition for preventing or treating adverse event of cholinesterase inhibitors, more specifically, it relates to a composition comprising as an active ingredient an antioxidant capable of preventing, alleviating, or treating adverse event caused by administration of a cholinesterase inhibitor in a therapeutically effective amount or more.

### BACKGROUND OF THE INVENTION

Dementia is a group of cognitive disorders characterized by memory loss, decline in intelligence, personality changes, and behavioral abnormalities. In other words, dementia is a degenerative neurological disease that is caused by irreversible dysfunction in the neural circuit network due to slow neuronal death that causes degenerative disorders of the central nervous system, and eventually leads to permanent loss of human functions. The cause of dementia has not yet been clearly identified, and since it has various etiological and pathophysiological factors, there is no treatment that can fundamentally treat dementia. However, it is known that choline acetyltransferase (ChAT), which synthesizes acetylcholine (ACh), is reduced by 20-30% in the brains of dementia patients compared to normal people, and the concentration of acetylcholine, a neurotransmitter, is reduced by 16-30%. As a result of the above research, acetylcholinesterase (AChE) inhibitors, which are acetylcholine degradation factors, are used as an indirect treatment method for the treatment of Alzheimer's disease, and Donepezil (trade name: Aricept), tacrine (trade name: Cognex), rivastigmine (trade name: Exelon), Galantamine (trade name: Reminyl) and the like belong.

Donepezil, an AChE inhibitor, is widely used for the treatment of mild to moderate dementia in Alzheimer's disease. AChE is an enzyme that hydrolyzes acetylcholine, one of the neurotransmitters that mediate the activity of parasympathetic nerves in the body, into choline and acetate, and is formed in the endoplasmic reticulum membrane and migrates to the cell membrane to perform its function. The enzyme is most distributed in the cholinergic nerve and its surroundings, especially in the neuromuscular junction, and is an important enzyme found in plasma, liver and other tissues. Therefore, systemic administration of AChE inhibitors, including donepezil, affects not only the target tissue but also other tissues, resulting in various adverse events such as diarrhea, nausea, dizziness, salivation, anxiety, tremor, lethargy, muscle spasms, decreased body temperature, extrapyramidal symptoms, miosis, which limits long-term use.

Therefore, there is a need to develop agents that reduce the adverse event of AChE inhibitors, including donepezil, so that they can be taken safely for long periods of time.

### DETAILED DESCRIPTION OF THE INVENTION

### TECHNICAL PROBLEM

Accordingly, as a result, the present inventor searched for disclosed substances that have been shown to be safe and effective in preventing, alleviating, or treating adverse event caused the administration of a cholinesterase inhibitor of a therapeutically effective amount or more, and found that antioxidants can have such effects, and thus completed the present invention.

Accordingly, an object of the present invention is to provide a pharmaceutical composition for preventing or treating adverse event of a cholinesterase inhibitor, comprising an antioxidant as an active ingredient.

In addition, an object of the present invention is to provide a pharmaceutical composition for preventing or treating adverse event of a cholinesterase inhibitor, consisting of an antioxidant as an active ingredient.

In addition, an object of the present invention is to provide a pharmaceutical composition for preventing or treating adverse event of a cholinesterase inhibitor, essentially consisting of an antioxidant as an active ingredient.

Another object of the present invention is to provide a food composition for preventing or improving adverse event of a cholinesterase inhibitor, comprising an antioxidant as an active ingredient.

Another object of the present invention is to provide use of an antioxidant for the preparation of a composition for treating adverse events of a cholinesterase inhibitors.

### TECHNICAL SOLUTION

In order to achieve the above object of the present invention, the present invention provides a pharmaceutical composition for preventing or treating adverse event of a cholinesterase inhibitor, comprising an antioxidant as an active ingredient.

In addition, in order to achieve the above object of the present invention, the present invention provides a pharmaceutical composition for preventing or treating adverse event of a cholinesterase inhibitor, consisting of an antioxidant as an active ingredient.

In addition, in order to achieve the above object of the present invention, the present invention provides a pharmaceutical composition for preventing or treating adverse event of a cholinesterase inhibitor, essentially consisting of an antioxidant as an active ingredient.

In order to achieve another object of the present invention, the present invention provides a food composition for preventing or improving adverse event of a cholinesterase inhibitor, comprising an antioxidant as an active ingredient.

In order to achieve another object of the present invention, the present invention provides use of an antioxidant for the preparation of a composition for treating adverse events of a cholinesterase inhibitors.

Hereinafter, the present invention will be described in detail.

The present invention provides a pharmaceutical composition for preventing or treating adverse event of a cholinesterase inhibitor, comprising an antioxidant as an active ingredient.

In the present invention, the "cholinesterase inhibitor" refers to a compound that inhibits the enzymatic degradation of the neurotransmitter acetylcholine and thus increases the duration and level of acetylcholine action in the synaptic cleft. Two enzymes are primarily responsible for the breakdown of acetylcholine, acetylcholinesterase and butyrylcholinesterase. The "cholinesterase inhibitor" comprises substances that inhibit or otherwise reduce the action of one or both of these enzymes.

In the compositions and methods of the present invention, cholinesterase inhibitors are considered pharmaceutically effective. As used herein, "pharmaceutically effective" means that the cholinesterase inhibitor is therapeutically useful in humans. For this reason, the term excludes cholinesterase inhibitors used as pesticides, such as aldicarb (2-methyl-2-(methylthio)propionaldehyde O-methylcarbamoyloxime), carbofuran (2,3-dihydro-2,2-dimethyl-7-benzofuranyl methylcarbamate), and carbaryl (1-naphthyl methylcarbamate), and cholinesterase inhibitors lethal enough to humans to be used as chemical weapons, such as sarin (2-(fluoro-methylphosphoryl)oxypropane), VX (S-[2-(diisopropylamino)ethyl ]-O-ethyl methylphosphonothioate), and soman (3-(fluoro-methyl-phosphoryl)oxy-2,2-dimethylbutane). Most cholinesterase inhibitors, pesticides and chemical weapons, are quasi-reversible or irreversible; Most of the pharmaceutically effective cholinesterase inhibitors are reversible.

Pharmaceutically effective cholinesterase inhibitors are well known in the art.
For example, 7-methoxytacrine, alvameline, ambenonium, anseculin, arecoline, cevimeline, citicoline, demacarium, donepezil, edrophonium, eptastigmine, fasciculin, heptyl-physostigmine, huperzine A and its analogs, icopezil , ipidacrine, linopirdine, metrifonate, milameline, neostigmine, pyridostigmine, norpyridostigmine, tacrine, physostigmine, rivastigmine, subcomeline, suronacrine, tacrine analogues, tacrine, talsaclidine, velnacrine, xanomeline, zifrosilone, itopride, acotiamide, huperzine, galanthamine and salts thereof may be included, but not limited thereto, and most preferably, the cholinesterase inhibitor may be donepezil.

Donepezil is an acetylcholinesterase (AChE) inhibitor having a structure represented by Formula 1 below and is used for the treatment of mild to moderate dementia in Alzheimer's disease. **In** Alzheimer's disease, in which cholinergic nervous system disorders in the brain have been reported, donepezil activates cholinergic neurons in the brain by increasing acetylcholine in the brain. Donepezil currently used commercially is in the form of a tablet (pill), and is being prescribed to patients with Alzheimer's disease in the form of an oral drug.

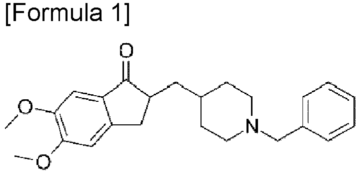

As the pharmaceutically acceptable salt of donepezil, an acid addition salt formed with a pharmaceutically acceptable free acid is useful. Acid addition salts are obtained from inorganic acids such as hydrochloric acid, nitric acid, phosphoric acid, sulfuric acid, hydrobromic acid, hydroiodic acid, nitrous acid or phosphorous acid and non-toxic organic acids such as aliphatic mono- and dicarboxylates, phenyl-substituted alkanoates, hydroxy alkanoates and alkanedioates, aromatic acids, aliphatic and aromatic sulfonic acids. These pharmaceutically non-toxic salts include sulfate, pyrosulfate, bisulphate, sulphite, bisulfite, nitrate, phosphate, monohydrogen phosphate, dihydrogen phosphate, metaphosphate, pyrophosphate chloride, bromide, ioda Id, fluoride, acetate, propionate, decanoate, caprylate, acrylate, formate, isobutyrate, caprate, heptanoate, propiolate, oxalate, malonate, succinate, suberate , sebacate, fumarate, maleate, butyne-1,4-dioate, hexane-1,6-dioate, benzoate, chlorobenzoate, methylbenzoate, dinitro benzoate, hydroxybenzoate, toxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycol Late, maleate, tartrate, methanesulfonate, propanesulfonate, naphthalene-1-sulfonate, naphthalene-2-sulfonate or mandelate may be used, but is not limited thereto.

**In** the present invention, the "antioxidant" is a substance that helps protect the human body from oxidative stress by removing active oxygen generated in the body, and its type is well known in the art. For example, the antioxidant may include N-acetylcysteine, ascorbic acid, alpha-lipoic acid, scopoletin, forsythin, isoferulic acid, gamma-oryzanol, trans-anethol, thioctic acid, cysteamine, gallic acid, salvianolic acid, vitamin E, lappaconitine, L-selenomethionine, selenium, coenzyme Q10, vitamin A, catechin, dantron etc., but is not limited thereto, preferably, it may be N-acetylcysteine, alpha-lipoic acid, daltron or a salt thereof, most preferably, it may be N-acetylcysteine or a salt thereof.

The N-acetylcysteine is a drug for inhibiting oxidative stress having the structure of Formula 2 below, which was developed about 50 years ago and is mainly used as an expectorant or used for the treatment of acetaminophen poisoning, which is an analgesic. N-acetylcysteine is known to exhibit antioxidant effects by directly reacting with peroxide, hydrogen peroxide, hydroxyl radical, etc. to reduce oxidative stress or indirectly increasing glutathione synthesis by providing cysteine, a raw material for glutathione biosynthesis. N-acetylcysteine has been used for decades in various clinical fields such as treatment of acute respiratory distress syndrome, treatment of acute pulmonary oxygen toxicity, and prevention of acute renal failure by contrast agents, and is known as a safe drug that does not cause adverse events.

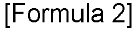

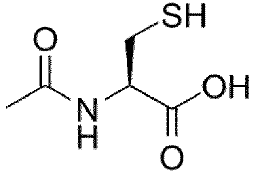

The adverse events of the cholinesterase inhibitor in the present invention are characterized by the administration of a cholinesterase inhibitor of a therapeutically effective amount or more.

The "therapeutically effective amount" refers to an amount of a cholinesterase inhibitor that relieves symptoms prior to treatment. Cholinesterase inhibitors are used as a treatment for dementia such as Alzheimer's, but the "symptoms" are not limited to dementia-related symptoms such as Alzheimer's, and may refer to all symptoms related to the therapeutic efficacy that cholinesterase inhibitors can exert.

In a preferred embodiment of the present invention, the "therapeutically effective amount" means a dose capable of relieving or treating symptoms before treatment by the administration of a cholinesterase inhibitor alone.

In another preferred embodiment of the present invention, the "therapeutically effective amount" means a dose capable of alleviating or treating Alzheimer's-related symptoms by the administration of a cholinesterase inhibitor alone. The Alzheimer's-related symptoms include, but are not limited to, psychobehavioral symptoms (cognitive function decline, memory decline, language ability decline, judgment decline, daily life performance decline, personality change, nervous behavior, depression, delusion, hallucination, aggression increase, sleep disorder, etc.), neurological disorders (stiffness, gait abnormality, etc.), and physical complications (urinary incontinence, infection, pressure sores, etc.).

In another preferred embodiment of the present invention, the "therapeutically effective amount" means a dose capable of alleviating or treating Alzheimer's-related symptoms by administration alone in a known usage of a cholinesterase inhibitor. The known usage may be interpreted as the same meaning as the clinically approved usage. For example, in the case of donepezil, a cholinesterase inhibitor, is approved for daily, once, 5 mg or 10 mg, oral administration or transdermal administration.

In a preferred embodiment of the present invention, the adverse events may refer to adverse events that may be caused by oral administration or transdermal administration, single administration or repeated administration of 0.05 mg/kg/day to 0.125 mg/kg/day or more of donepezil.

In another preferred aspect of the present invention, the adverse events may refer to adverse events that may occur when 5 mg, 10 mg, or 23 mg or more of donepezil is administered daily, once, orally or transdermally, or by single or repeated administration.

In the present invention, the " adverse events " refers to all abnormal symptoms other than the therapeutic effect that may occur by administration of a cholinesterase inhibitor in a therapeutically effective amount or more, and the type is not particularly limited.

For example, the types of adverse reactions reported to be causally related to the administration of donepezil are as follows:
1) Syncope, bradycardia, cardiac block, QT interval prolongation, myocardial infarction, heart failure, asthenia, hypothermia, urinary incontinence, rash, miosis
2) Anorexia, diarrhea, nausea, abdominal pain, nausea, peptic ulcer, salivation (drooling, flaccidity), perforated duodenal perforation, gastrointestinal bleeding
3) Hepatitis, liver dysfunction, jaundice
4) Insomnia, hallucinations, drowsiness, dizziness, tremor, stupor, cerebral seizures (epilepsy, convulsions, etc.), cerebral hemorrhage, cerebrovascular disorders
5) Extrapyramidal disorders: motor dysfunction, ataxia, decreased voluntary movement, dystonia, tremor, gait disorder, abnormal posture, speech disorder
6) Symptoms such as ataxia, extreme muscle stiffness, dysphagia, tachycardia, bradycardia, blood pressure fluctuations (hypertension, hypotension), sweating (tremors), and pallor may occur, and these symptoms are usually accompanied by fever.
7) Since rhabdomyolysis may occur, muscle pain, weakness, increased CK (CPK) in blood and urine,
8) Dyspnea
9) Acute pancreatitis
10) Acute renal failure
11) Sudden death of unknown cause

In the present invention, the antioxidant comprised in the pharmaceutical composition may be comprised in an amount of 30 to 200 times the weight of the cholinesterase inhibitor taken by the subject to be administered, preferably, it may be comprised 50 to 150 times, and most preferably 60 to 120 times.

In the present invention, the composition is administered for the purpose of preventing, alleviating, or treating adverse events of administration of a cholinesterase inhibitor, and may be administered before, simultaneously with or after administration of the cholinesterase inhibitor.

In the present invention, the term "prevention or treatment of adverse events " may be interpreted as including blocking the occurrence of adverse events, alleviating adverse events that have occurred, reducing the frequency of adverse events, delaying the occurrence of adverse events, or shortening the period of occurrence of adverse events.

The composition may be administered orally or parenterally. Parenteral administration may include, but is not limited to, intravenous, intramuscular, intraarterial, intramedullary, intrathecal, intrathecal, intracardiac, transdermal, subcutaneous, intrathecal, intracerebroventricular (subventricular zone), intracerebral, intraperitoneal, intranasal, enteral, topical, sublingual, or rectal.

The pharmaceutical composition according to the present invention may comprise only a pharmaceutically effective amount of an antioxidant or additionally comprise a pharmaceutically acceptable carrier. The "pharmaceutically effective amount" refers to an amount that exhibits a higher reaction than that of the negative control, preferably, it means an amount sufficient to prevent, ameliorate, or treat adverse events by administering an antioxidant in treating or preventing adverse events of cholinesterase inhibitors.

The pharmaceutical composition of the present invention may be formulated in various ways according to the route of administration by a method known in the art together with a pharmaceutically acceptable carrier. By "pharmaceutically acceptable" is meant a composition that is physiologically acceptable and, when administered to humans, non-toxic, which does not interfere with the action of the active ingredient and does not normally cause gastrointestinal disturbances, allergic reactions such as dizziness, or similar reactions. The carriers include solvents of all kinds, dispersion media, oil-in-water or oil-in-water emulsions, aqueous compositions, liposomes, microbeads and microsomes. Pharmaceutically acceptable carriers for inclusion in the compositions are those conventionally utilized in pharmaceutical formulations, including, but not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil. As other pharmaceutically acceptable carriers, reference may be made to those described in the following literature (Remington's Pharmaceutical Sciences, 19th ed., Mack Publishing Company, Easton, PA, 1995).

In addition to the above ingredients, the pharmaceutical composition may further comprise lubricants, wetting agents, sweeteners, flavoring agents, emulsifiers, suspending agents, preservatives, and the like. Specifically, for oral administration, a binder, an active agent, a disintegrant, an excipient, a solubilizer, a dispersant, a stabilizer, a suspending agent, a color or flavor, and the like may be used, for injectables, a mixture of buffers, preservatives, non-currency agents, solubilizers, isotonic agents, stabilizers, and the like may be used, for topical administration, a base, excipients, lubricants, preservatives, etc. may be used.

In addition, the compositions of the present invention can be used in the form of conventional pharmaceutical preparations. Parenteral preparations include sterile aqueous solutions, non-aqueous solutions, suspensions, emulsions or lyophilized preparations, injections, transdermal preparations, nasal inhalations, and the like, for oral administration, it may be formulated as tablets, troches, capsules, elixirs, suspensions, syrups, or wafers. In the case of injectables, they can be prepared in single-dose ampoules or multiple-dose formulations. In the case of injectables, they must be sterile and protected from contamination by microorganisms such as bacteria and fungi. Examples of suitable carriers for injectables include, but are not limited to, solvents or dispersion media comprising water, ethanol, polyols (e.g., glycerol, propylene glycol, and liquid polyethylene glycol), mixtures thereof, and/or vegetable oils. More preferably, suitable carriers include Hanks' solution, Ringer's solution, phosphate buffered saline (PBS) containing triethanolamine, or isotonic solutions such as sterile water for injection, 10% ethanol, 40% propylene glycol, and 5% dextrose. To protect the injectable from microbial contamination, the injectable may additionally contain various antibacterial and antifungal agents such as parabens, chlorobutanol, phenols, sorbic acid, thimerosal, and the like. In addition, the injectable may further comprise an isotonic agent such as sugar or sodium chloride in most cases.

Furthermore, the pharmaceutical compositions of the present invention may be administered by any device that allows the active substance to be transported to the target site. Preferred modes of administration and formulations include intravenous, subcutaneous, intradermal, intramuscular, or drip injections. The injectable formulation can be prepared using an aqueous solvent, such as saline or intravenous solution, a non-aqueous solvent, such as a vegetable oil, a higher fatty acid ester (e.g., oleic acid ethyl), an alcohol (e.g., ethanol, benzyl alcohol, propylene glycol, or glycerin), etc, and included in pharmaceutical carriers such as stabilizers to prevent spoilage (e.g., ascorbic acid, sodium hydrogen sulfite, sodium pyrosulfite, BHA, tocopherols, EDTA, etc.), emulsifiers, buffers to adjust pH, and preservatives to inhibit microbial growth (e.g., phenylmercury nitrate, thimerosal, benzalkonium chloride, phenol, cresol, benzyl alcohol, etc.). A method of treating or preventing a neurodegenerative disease using a composition of the present invention comprises administering an effective amount (pharmaceutically effective amount) of a therapeutic composition of the present invention to a subject in need thereof. The pharmaceutically effective amount can be readily determined by those skilled in the art based on factors well known in the medical field, such as the type of disease, the patient's age, weight, health, gender, the patient's sensitivity to the drug, the route of administration, the method of administration, the number of doses, the duration of treatment, and the drugs used in combination or concurrently.

In addition, the pharmaceutical compositions of the present invention can be formulated using methods known in the art to provide a rapid, sustained, or delayed release of the active ingredient after administration to a mammal.

The total effective amount of a composition of the present invention may be administered to a patient in a single dose, or in a fractionated treatment protocol where multiple doses are administered over an extended period of time. The pharmaceutical compositions of the present invention can have different amounts of the active ingredient depending on the severity of the disease. It should be noted that the effective dose of the pharmaceutical composition to a patient will be determined not only by the method of formulation, route of administration, and number of treatments, but also by a variety of factors, including the age, weight, health status, gender, severity of the disease, diet, and excretion rate of the patient, and in light of these considerations, one having ordinary skill in the art will be able to determine the appropriate effective dose of the composition of the invention. The pharmaceutical compositions according to the present invention are not particularly limited in their formulation, route of administration and method of administration as long as they exhibit the effects of the present invention.

The present invention also provides a food composition for preventing or improving adverse event of a cholinesterase inhibitor, comprising an antioxidant as an active ingredient.

The food compositions of the present invention include all forms of functional foods, nutritional supplements, health foods, and food additives.

Food compositions of the above types can be prepared in various forms according to conventional methods known in the art. By way of example, but not limitation, the cholinesterase inhibitor and antioxidant can be liquefied, granulated, encapsulated, and powdered for consumption in the form of teas, juices, and drinks. In addition, donepezil and N-acetylcysteine can be formulated into compositions by mixing them with the active ingredients of the disclosure known to be effective against infectious diseases. Furthermore, In addition, cholinesterase inhibitors and antioxidants can be added to beverages (including alcoholic beverages), fruits and their processed products (e.g., canned, bottled, jam, marmalade, etc.), fish, meat and their processed products (e.g., ham, sausage corned beef, etc.), breads and noodles (e.g., udon, soba, ramen, spaghetti, macaroni, etc.), fruit juices, various beverages, cookies, malt, dairy products (e.g., butter, cheese, etc.), edible vegetable oils, margarine, vegetable proteins, retort foods, frozen foods, various condiments (e.g., miso, soy sauce, sauces, etc.), etc. including, but not limited to, functional foods. In addition, cholinesterase inhibitors and antioxidants can be formulated in powder or concentrate form for use as additives.

The content of the antioxidant in the food composition of the present invention is not limited thereto, but is preferably 0.1 to 90% by weight of the finally prepared food. More preferably, a food composition comprising the antioxidant of the present invention as an active ingredient, in particular, can be prepared in the form of a health functional food or dietary supplement by mixing with an active ingredient known to be effective in alleviating the adverse event of cholinesterase inhibitors.

The present invention also provides use of an antioxidant for the preparation of a composition for treating adverse events of a cholinesterase inhibitors.

The term "treating" in the present invention refers comprehensively to ameliorating a side effect of a cholinesterase inhibitor or a symptom resulting from a disease, which may include curing, substantially preventing, or improving a condition, and may include, but is not limited to, alleviating, treating, or preventing one or most symptoms resulting from a disease.

As used herein, the term "comprising" is used interchangeably with "including" or "characterized by" and does not exclude additional components or steps in a composition or method according to the present invention that are not specifically mentioned. The term "consisting of" also means to exclude additional elements, steps, or components not specifically mentioned. The term "essentially consisting of" means that the scope of a composition or method may include, in addition to the listed substances or steps, substances or steps that do not materially affect its basic characteristics.

### ADVANTAGEOUS EFFECT

Since the pharmaceutical composition of the present invention containing an antioxidant as an active ingredient exhibits an effect of preventing, alleviating, or treating adverse events that may occur due to the administration of a cholinesterase inhibitor in a therapeutically effective amount or more, not only can it mitigate the risk of adverse event caused by the administration of a cholinesterase inhibitor in a therapeutically effective amount, but it can also be very useful for the treatment of patients who require administration of a cholinesterase inhibitor of a therapeutically effective amount or more.

### BRIEF DESCRIPTION OF THE DRAWINGS

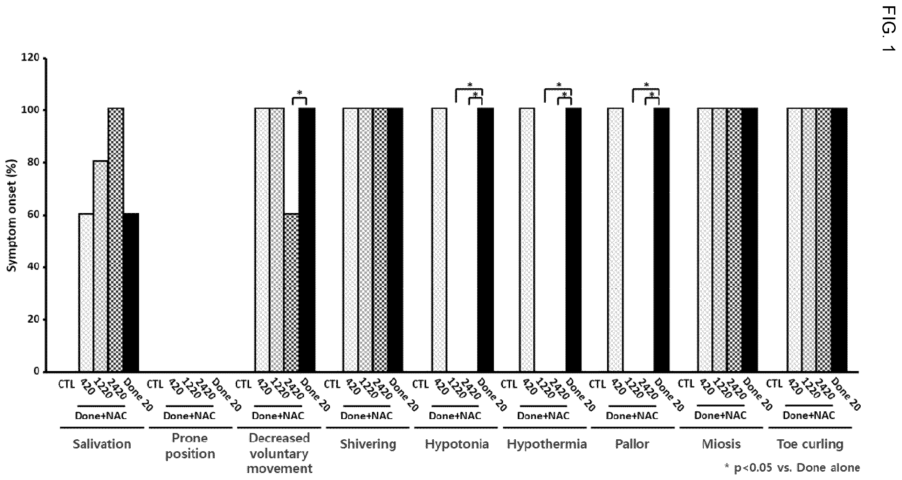

FIG. 1 shows the results of measuring the incidence of adverse events caused by donepezil in female rats to which either donepezil alone or a combination of donepezil and NAC was orally administered once a day for 4 weeks. The control group was administered sterile water for injection. Adverse events of hypotonia, hypothermia, and pallor were reduced in rats administered with the combination of 20 mg/kg of donepezil and 1200 and 2400 mg/kg of NAC compared to 20 mg/kg of donepezil alone. The decrease in spontaneous movement was reduced only in rats administered with the combination of 20 mg/kg of donepezil and 2400 mg/kg of NAC.

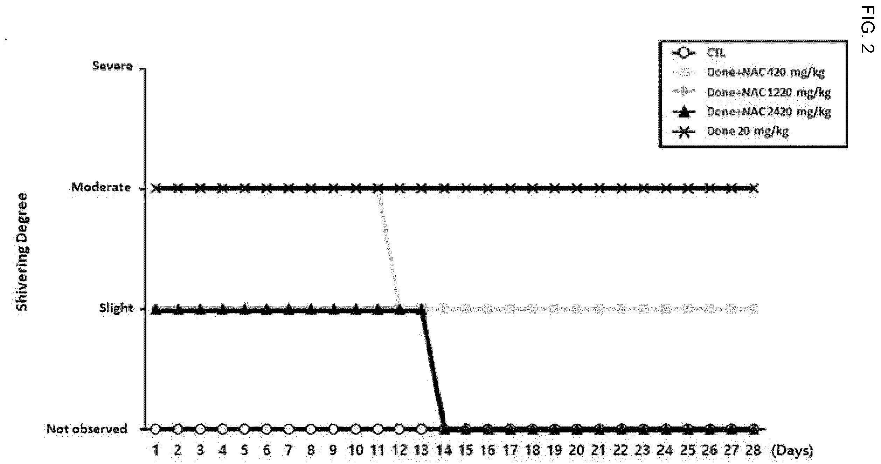

FIG. 2 shows the results of measuring the degree and duration of tremor among adverse events caused by AChE inhibitors in female rats to which either donepezil alone or a combination of donepezil and NAC was orally administered once a day for 4 weeks.

### MODE FOR CARRYING OUT INVENTION

Hereinafter, the present invention is described in detail by the following embodiments. However, the following embodiments are intended to illustrate the invention, and the invention is not limited by them.

### Effect of improving adverse events of cholinesterase inhibitors according to antioxidant administration

The purpose of this study was to compare the degree of adverse events associated with the group administered with 20 mg/kg of donepezil after administering a combination drug at a concentration of 20 mg/kg of donepezil, a cholinesterase inhibitor, and 400 mg/kg, 1200 mg/kg, 2400 mg/kg of N-acetylcysteine (NAC), an antioxidant.

Specifically, sterile water for injection, 20 mg/kg of donepezil, or a combination of donepezil and NAC (420 mg/kg, 1220 mg/kg, 2420 mg/kg) were orally administered to female Sprague-Dawley (SD) rats (Orient Bio Co., Ltd.) once a day for 4 weeks. The type, expression, and severity of adverse events related to the cholinesterase inhibitor that occurred during the administration period were observed at least once a day and recorded for each subject.

The types of adverse events of donepezil that may occur in rats and the types of adverse events that may occur in humans may be related as shown in Table 1 below.

**Table 1**

| **rats** | **human** |
|---|---|
| Salivation | salivation |
| prone position | extrapyramidal disorder, asthenia, drowsiness |
| decrease in locomotor activity | extrapyramidal disorder, asthenia, drowsiness |
| shivering | extrapyramidal disorder, tremor |
| hypotonia | extrapyramidal disorder, asthenia, drowsiness |
| hypothermia | hypothermia |
| Pallor | Nausea, bradycardia, hypotension |
| miosis | miosis |
| curled toes | extrapyramidal disorder, muscle spasm |

As shown in FIG. 1, in female rats, adverse events such as hypotonia, hypothermia, and pallor were reduced in rats administered with the combination of 20 mg/kg of donepezil and 1200 and 2400 mg/kg of NAC compared to 20 mg/kg of donepezil alone. In addition, the adverse event of decreased locomotor activity was reduced in rats administered with the combination of 20 mg/kg of donepezil and 2400 mg/kg of NAC. Decreased locomotor activity and hypotonia are common adverse events in rats caused by AChE inhibitor administration, and are related to extrapyramidal symptoms and asthenia in humans. In addition, since hypothermia is related to decreased body temperature, an adverse event of AChE inhibitors, pallor is related to nausea, bradycardia, hypotension, etc., it was confirmed that adverse events caused by cholinesterase inhibitors were reduced in the combination drug.

As shown in FIG. 2, the degree and duration of shivering were reduced in rats administered with the combination of 20 mg/kg of donepezil and 400, 1200, and 2400 mg/kg of NAC compared to 20 mg/kg of donepezil alone in female rats. Shivering is a common the adverse event of cholinesterase inhibitor administration, it was confirmed that the adverse events caused by cholinesterase inhibitors were reduced in the combination drug, as it was associated with extrapyramidal symptoms and tremor in the human body.

### INDUSTRIAL APPLICABILITY

Since the pharmaceutical composition of the present invention containing an antioxidant as an active ingredient exhibits an effect of preventing, alleviating, or treating adverse events that may occur due to the administration of a cholinesterase inhibitor in a therapeutically effective amount or more, industrial applicability is very high because not only can it mitigate the risk of adverse event caused by the administration of a cholinesterase inhibitor in a therapeutically effective amount, but it can also be very useful for the treatment of patients who require administration of a cholinesterase inhibitor of a therapeutically effective amount or more.

## Claims

1. A pharmaceutical composition for preventing or treating adverse event of a cholinesterase inhibitor, comprising an antioxidant as an active ingredient.

2. The pharmaceutical composition according to claim 1, wherein the antioxidant is at least one selected from the group consisting of N-acetylcysteine, ascorbic acid, alpha-lipoic acid, scopoletin, forsythin, isoferulic acid, gamma-oryzanol, trans-anethole, thioctic acid, cysteamine, gallic acid, salvianolic acid, vitamin E, lappaconitine, selenium, coenzyme Q10, vitamin A, catechin, dantron and L-selenomethionine or a salt thereof.

3. The pharmaceutical composition according to claim 1, wherein the cholinesterase inhibitor is at least one selected from the group consisting of 7-methoxytacrine, alvameline, ambenonium, anseculin, arecoline, cevimeline, citicoline, demacarium, donepezil, edrophonium, eptastigmine, fasciculin, heptyl-physostigmine, huperzine A and its analogs, icopezil, ipidacrine, linopirdine, metrifonate, milameline, neostigmine, nomeostigmine, pyridostigmine, norpyridostigmine, tacrine, physostigmine, rivastigmine, subcomeline, suronacrine, tacrine analogues, tacrine, talsaclidine, velnacrine, xanomeline, itopride, acotiamide, huperzine, galanthamine and zifrosilone or a salt thereof.

4. The pharmaceutical composition according to claim 1, wherein the adverse event is caused by administration of a cholinesterase inhibitor in a therapeutically effective amount or greater.

5. The pharmaceutical composition according to claim 4, wherein the therapeutically effective amount is 0.05 mg/kg to 0.125 mg/kg.

6. The pharmaceutical composition according to claim 4, wherein the antioxidant is comprised in an amount of 30 to 200 times compared to the therapeutically effective amount of the cholinesterase inhibitor.

7. The pharmaceutical composition according to claim 1, wherein the composition is administered before, simultaneously with, or after administration of the cholinesterase inhibitor.

8. The pharmaceutical composition according to claim 1, wherein the adverse event is selected from the group consisting of extrapyramidal symptoms, asthenia, hypothermia, shivering, convulsions, pallor, sickness, bradycardia, hypotension, anorexia, diarrhea, nausea, insomnia, hallucinations, drowsiness, dizziness, incontinence, rash, miosis and drooling.

9. The pharmaceutical composition according to claim 8, wherein the extrapyramidal symptom is selected from the group consisting of motor dysfunction, ataxia, decreased spontaneous movement, dystonia, tremor, gait disorder, abnormal posture and speech disorder.

10. A food composition for preventing or improving adverse event of a cholinesterase inhibitor, comprising an antioxidant as an active ingredient.

11. Use of an antioxidant for the preparation of a composition for treating adverse events of a cholinesterase inhibitors.
